# EUROPÄISCHE PATENTANMELDUNG

(11) **EP 0 588 228 A1**
(43) Veröffentlichungstag der Anmeldung: **23.03.1994**
(21) Anmeldenummer: 93114409.1
(22) Anmeldetag: 08.09.1993
(51) Int. Cl.: A61L 2/26, A61B 19/02, B65D 43/08

(54) **Sterilisierfähiges Behältnis für einzelne medizinische und chirurgische Bestecke und Instrumente**

(30) Priorität: 12.09.1992 DE 9212315 U; 15.06.1993 DE 9308769 U
(71) Anmelder: Ritter, Ralf, 86836 Untermeitingen (DE)
(72) Erfinder: Ritter, Ralf, 86836 Untermeitingen (DE)
(74) Vertreter: Gallo, Wolfgang, Dipl.-Ing. (FH)

(57) **Zusammenfassung**

Mehrfach sterilisierfähiges Behältnis für einzelne medizinische und chirurgische Bestecke, Instrumente und Geräte in Form einer steifen Box aus sterlilisiertemperaturbeständigem Kunststoff, bestehend aus einem Unterteil (1) mit Seitenwänden und einem darauf aufsetzbaren und keimdicht abschließenden Deckel (2), wobei in Unterteilseitenwandbereichen mit einer keimdichten, jedoch für Sterilisierdampf bzw. Sterilisiergas durchlässigen Filterschicht (3) abgedeckte Wanddurchbrüche (11) vorgesehen sind, wobei der Deckel für eine die Sterilität überprüfbar gewährleistende gesicherte Schließbarkeit auf dem Unterteil besonders ausgebildet ist und Maßnahmen zur Austauschbarkeit der Filterschicht getroffen sind.

## Beschreibung

Das Deutsche Gebrauchsmuster 91 05 867 hat ein sterilisierfähiges Behältnis für einzelne medizinische und chirurgische Bestecke, Instrumente und Geräte zum Gegenstand, das als mehrfach sterilisierfähige steife Box aus sterilisiertemperaturbeständigem Kunststoff ausgebildet ist, die aus einem Unterteil und einem damit zusammenwirkenden und keimdicht abschließenden Deckel besteht und mittels einer keimdichten, jedoch für Sterilisierdampf bzw. Sterilisiergas durchlässigen Filterschicht abgedeckte Wanddurchbrüche aufweist.

Die Wanddurchbrüche sind in den Seitenwänden des Unterteils gebildet, und die Eilterschicht ist zwischen diesen Seitenwänden und einem in das Unterteil eingesetzten Einsatz eingebettet, der mit den Durchbrüchen der Unterteilseitenwände entsprechenden Durchbrüchen versehen ist. Der Deckel ist mit Scharnieren am Unterteil angelenkt und in geschlossenem Zustand durch einen Etikettenaufkleber versiegelbar.

Der vorliegenden Erfindung liegt die Aufgabe zugrunde, ein solches sterilisierfähiges Behältnis im Hinblick auf seine praktischen Handhabungseigenschaften, die Sicherheit der Bewahrung des Sterilzustands bis zum Gebrauch des darin enthaltenen Bestecks, Instruments oder Geräts, und der leichten Überprüfbarkeit des Sterilzustands konstruktiv zu verbessern.

Diese Aufgabe wird gemäß der Erfindung durch das im Anspruch 1 angegebene und in den Unteransprüchen weiter ausgestaltete Behältnis gelöst.

Die mit der erfindungsgemäßen Gestaltung erreichten Vorteile werden im Zuge der nachstehenden Beschreibung eines Ausführungsbeispiels unter Bezugnahme auf die anliegenden Zeichnungen im einzelnen darlegt. In den Zeichnungen zeigt:
- Fig. 1: einen Längsschnitt durch ein Behältnis nach der Erfindung in geschlossenem Zustand
- Fig. 2: einen Längsschnitt ähnlich Fig. 1 unmittelbar vor dem Verschließen des Deckels,
- Fig. 3: eine Frontansicht des geschlossenen Behältnisses ohne Etikett mit Schnittdarstellung des Seitenwandbereichs,
- Fig. 4: eine Frontansicht des geschlossenen Behältnisses mit eingesetztem Etikett,
- Fig. 5: in gesonderter Darstellung das in Fig. 4 in das Behältnis eingesetzte Sicherungsetikett,
- Fig. 6: eine perspektivische Darstellung einer weiteren Ausführungsform einer Sterilisierbox nach der Erfindung mit geschlossenem und verriegeltem Deckel,
- Fig. 7: in perspektivischer Darstellung den Aufbau einer Längsseitenwand des Sterilisierbox-Unterteils, der Box nach Fig. 6,
- Fig. 8: den Aufbau des zweiteiligen Deckels und den Deckelverriegelungsmechanismus, und
- Fig. 9: die Sicherung der geschlossenen Sterilsierbox mittels eines Sicherungsetiketts.

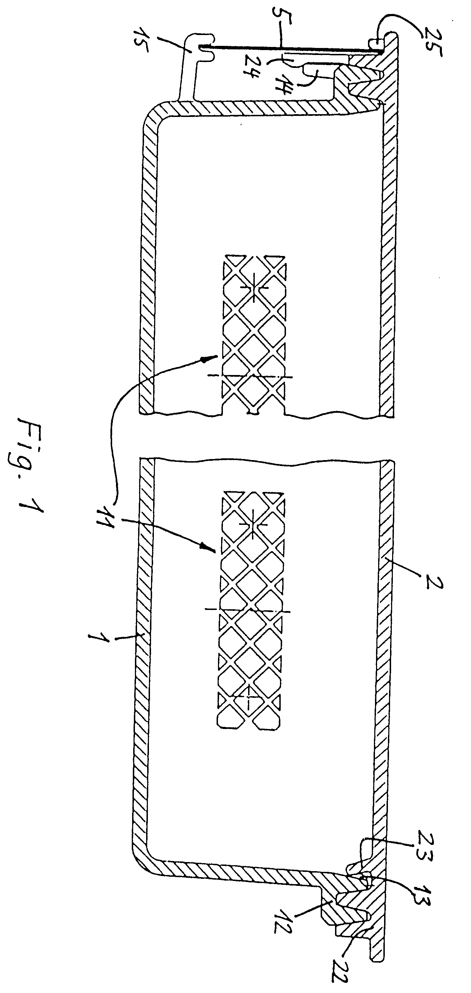

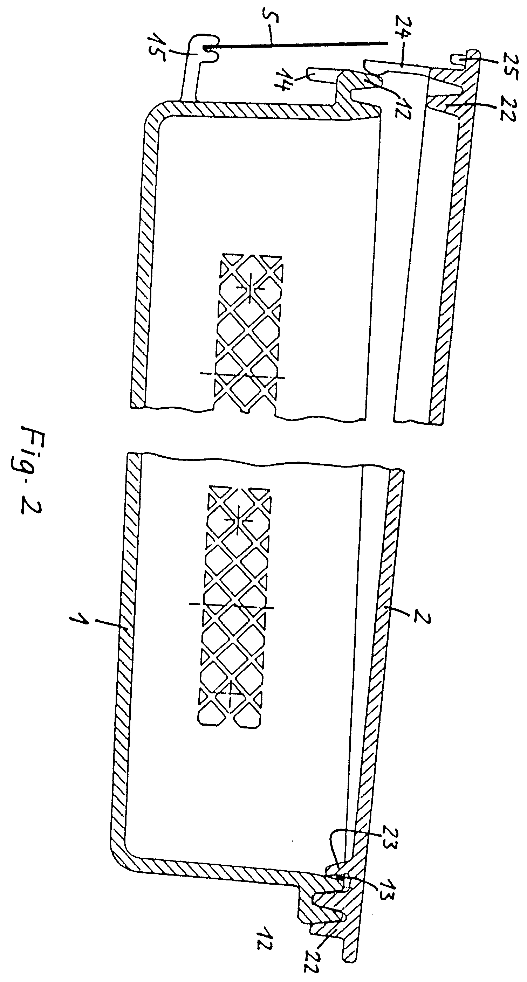

Damit die hier interessierenden Einzelheiten erkennbar werden, sind alle Darstellungen der Fig. 1 bis 5 etwa im Maßstab 2 : 1 gehalten, also in vierfacher natürlicher Größe, wobei die Darstellungen in den Fig. 1 und 2 durch Herausbrechen des Mittelteils axial verkürzt sind.

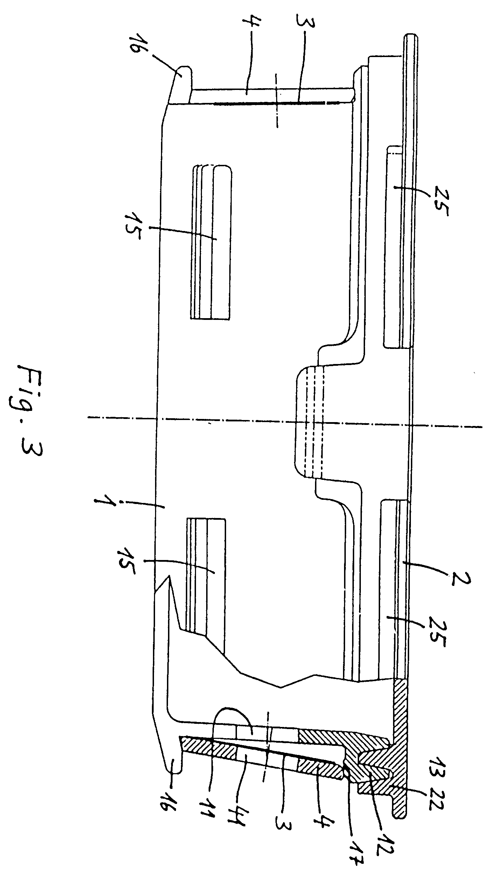

Fig. 1 zeigt ein Behältnis nach der Erfindung, nämlich eine Sterilisierbox, im Längsschnitt. Sie besteht aus einem Unterteil 1 und einem darauf aufgesetzten Deckel 2. Die längsverlaufenden Seitenwände sind jeweils mit einer Anzahl gitterartiger Durchbrechungsbereiche 11 versehen, die, wie aus Fig. 3 ersichtlich ist, jeweils von außen mit einer keimdichten, aber sterilisiergasdurchlässigen Filterschicht 3 abgedeckt sind, die mittels jeweils einer an der betreffenden Längsseitenwand außen eingerasteten Halteplatte 4 mit entsprechenden Durchbrechungsbereichen 41 gehaltert ist.

Das Unterteil 1 weist an seinem oberen Rand ein umlaufendes Doppelstegprofil 22 am Unterseitenrandbereich des Deckels 2 eingreift und damit zusammen einen keimdichten Verschluß herstellt.

Wie aus den Schnittdarstellungen nach den Fig. 1 und 2 hervorgeht, hat der Deckel 2 an seiner hinteren Schmalseite außer dem Doppelstegprofil 22 noch eine innenseitig vorgesetzte, schräg rückwärts orientierte Rippe 23, die einen etwa der Innenseite des Doppelstegprofils 12 an der hinteren Schmalseite des Unterteils 1 hintergreift, wenn der Deckel geschlossen ist, wie in Fig. 1 dargestellt ist, so daß dann ein Abheben des Deckels vom Unterteil am hinteren Ende der Box ausgeschlossen ist. Zum Aufsetzen des Deckels wird dieser daher in Schräglage (siehe Fig. 2) zunächst mit einer hinteren Schmalseite aufgesetzt und dann mit seiner vorderen Schmalseite nach unten abgekippt, wobei die Schrägrippe 23 den Vorsprung 13 um- und hintergreift.

An seiner vorderen Schmalseite trägt der Deckel mittig eine als einstückige Verlängerung des äußeren Stegs seines dortigen Doppelstegprofils 22 angeformte, wegen ihrer Länge und den Eigenschaften des Kunststoffmaterials federelastische Rastlasche 24, welche eine komplementär an der entsprechenden Stelle des Doppelstegprofils 12 des Unterteils angeformte Nase 14 einrastend hintergreift, wenn der Deckel vollständig geschlossen auf dem Unterteil sitzt (siehe Fig. 1). Beim Schließvorgang (siehe Fig. 2) gleitet diese Rastlasche 24, wie dargestellt, über die Außenseite des Doppelstegprofils 12 und die daran angeformte Rastnase 14.

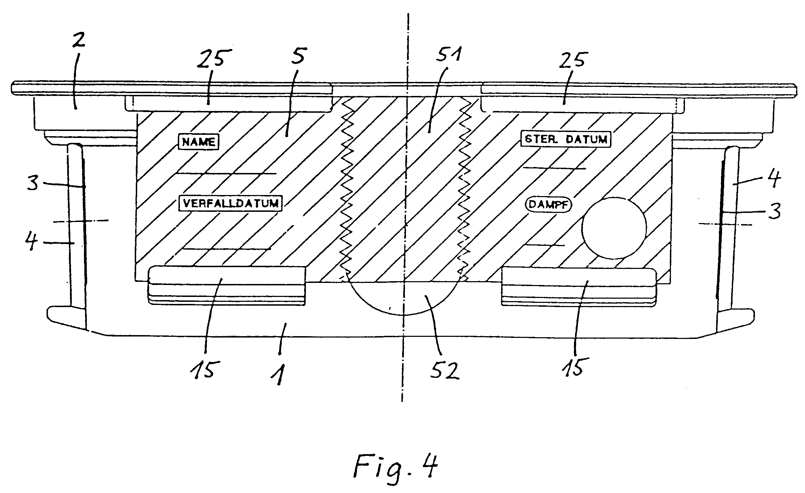

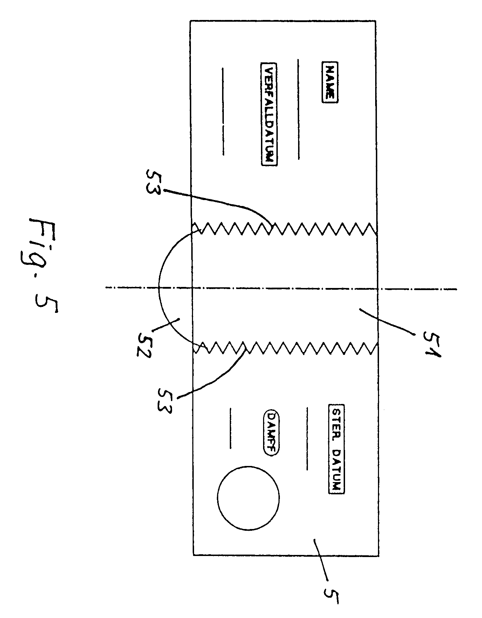

Am vorderen Ende der Box, also an den vorderen Schmalseiten von Unterteil 1 und Deckel 2, sind leistenartige Halteprofile 15 bzw. 25 angeformt, die Haltenuten zum Einstecken eines Sicherungsetiketts 5 bilden. Das Sicherungsetikett 5 besteht beispielsweise aus steifem Karton und ist in den Fig. 1 und 2 in Seitenansicht in den Fig. 4 und 5 in Frontansicht dargestellt. Beim Aufsetzen des Deckels wird das Sicherungsetikett 5 zunächst mit seiner Unterkante in die Halteleiste 15 des Unterteils 1 eingesteckt und beim Schließen des vorderen Deckelendes (siehe Fig. 2) so an die Haltelasche 24 angelehnt, daß die Oberkante des Sicherungsetiketts beim vollständigen Niederdrücken des Deckels in seine Verschlußstellung in die Haltenut der oberen Halteleiste 25 des Deckels 2 hineingleitet.

Wie in der linken Hälfte der Fig. 3 gestrichelt dargestellt ist, ist die Aufnahmenut für das Sicherungsetikett an den äußeren Enden der oberen Halteleiste 25 geschlossen, so daß das eingesetzte Sicherungsetikett bei geschlossenem Deckel gegen seitliches Herausschieben gesichert ist. Das Sicherungsetikett 5 verdeckt die Rastlasche 24 des Deckels, so daß nach dem Schließen der Box die Rastlasche nicht mehr zugänglich und deshalb die Box nicht mehr zu öffnen ist, ohne das Sicherungsetikett zu zerstören. Dies geht deutlich aus den Fig. 3 und 4 hervor, von denen Fig. 3 die vordere Stirnansicht der Box bei geschlossenem Deckel mit der Sicherungslasche 24 bei weggelassenem Sicherungsetikett darstellt, während Fig. 4 eine ähnliche Darstellung mit eingesetztem Sicherungsetikett zeigt, das nun die Rastlasche verdeckt.

Fig. 5 zeigt zur Verdeutlichung des Sicherungsetikett allein in Ansicht. Dieses trägt verschiedene Daten, die hier nicht im einzelnen interessieren, und weist eine mittige Aufreißlasche 51 mit einem unteren halbrunden Ansatz 52 zum leichteren Ergreifen mit den Fingern auf. Diese Aufreißlasche 51 ist, wie dargestellt, beiderseits durch Zickzack-Vorstanzungen mit den beiderseitigen übrigen Etikettbereichen verbunden und wird, wenn die Box geöffnet werden soll, an dem Ansatz 52 ergriffen und abgerissen. Dadurch wird die Rastlasche 24 des Deckels zugänglich und der Deckel kann geöffnet werden.

Aus Fig. 3 ist außerdem ersichtlich, wie die Halteplatten 4 an den beiden Längsseitenwänden des Unterteils zu Fixierung der Filterschichten 3 einrastbar gehaltert sind. Außen entlang der Unterseite jeder Längsseitenwand verläuft ein Haltesteg 16, der eine schräge Sitzfläche für die entsprechend abgeschrägte Unterkante der Halteplatte 4 bildet, und oben ist an der Unterseite ein leichter Vorsprung 17 gebildet, der mit der abgerundeten Oberkante der Halteplatte 4 zusammenwirkt. Die Halteplatte 4 mit der vorgesetzten, vorzugsweise durch Kleben oder dergleichen darauf fixierten Filterschicht 3, wird zunächst mit seiner Unterkante schräg auf die untere Halteleiste 16 angesetzt, wie in der rechten Hälfte der Fig. 3 im Schnitt dargestellt, und sodann mit seinem Oberkantenbereich einwärts gedrückt, wobei seine Oberkante federelastisch hinter den Vorsprung 17 einrastet.

Die erfindungsgemäße Box bringt, wie aus der vorstehenden Beschreibung hervorgeht, zwei wesentliche handhabungstechnische Vorteile:
Erstens ist es nicht möglich, den Deckel der Box ohne Zerstörung des Sicherungsetiketts, also unbemerkt, zu öffnen und damit den Sterilzustand zu zerstören, oder mit anderen Worten, das unbeschädigte Sicherungsetikett an der geschlossenen Box signalisiert das Vorhandensein des orginal-sterilen Zustands.

Die an der Außenseite mittels einrastbarer Halteplatte 4 gehalterten Filterschichten 3 ergeben eine Innenwandfläche der Box, die frei von Einsätzen oder dgl. ist, wodurch die praktische Handhabung beim Einlegen und Herausnehmen der medizinischen Gerätschaften erleichtert wird. Dennoch sind die Filterschichten wegen der Rasthalterung der Halteplatten 4 bei Bedarf leicht auswechselbar.

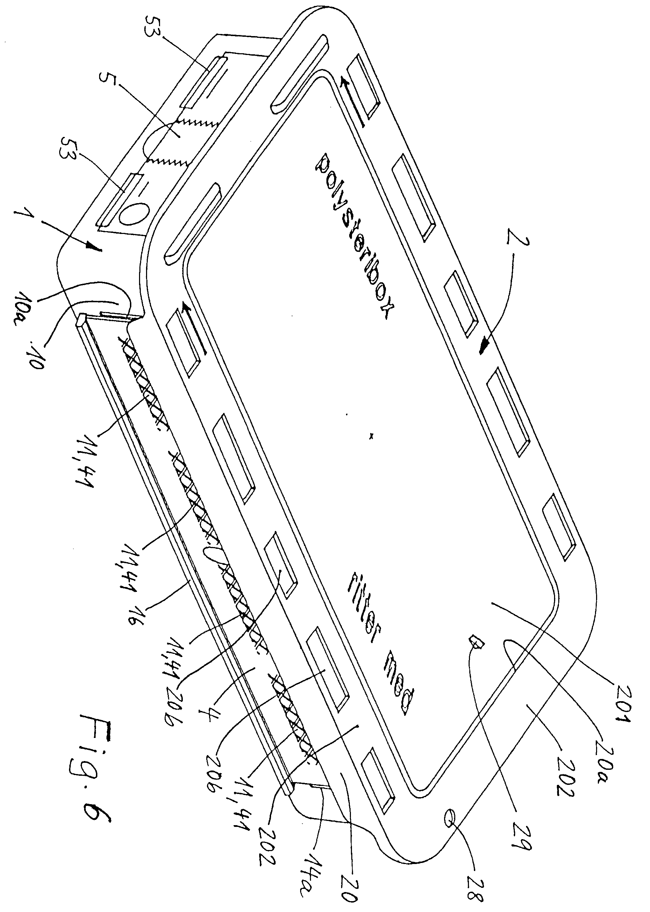

Die Fig. 6 bis 9 zeigen eine weitere Ausführungsform des Behältnisses nach der Erfindung, die sich im wesentlichen durch eine andere, zweiteilige Deckelkonstruktion von der Ausführungsform nach den Fig. 1 und 5 unterscheidet, die noch weitere Vorteile bringt, insbesondere hinsichtlich der Sicherung des Sterilzustands bis zum Gebrauch.

In Fig. 6 ist ein Behältnis nach der weiteren Ausführungsform der Erfindung im kompletten geschlossenen Zustand dargestellt. Die beiden längeren Seitenwände des Unterteils 1 sind wiederum jeweils mit einer Anzahl gitterartiger Durchbrechungsbereiche 11 versehen, die eine keimdichte, aber sterilisiergasdurchlässige Filterschicht 3 enthalten.

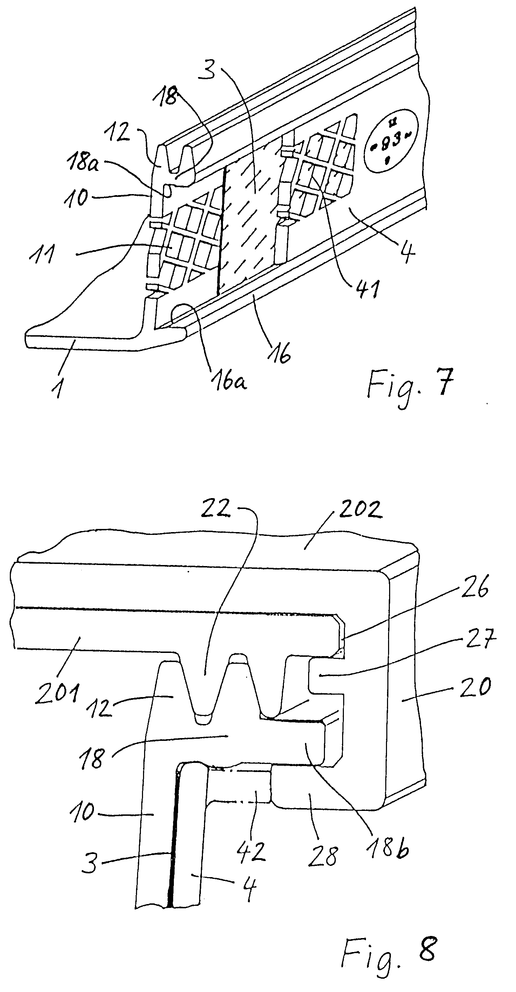

Der Aufbau der mit den gitterartigen Durchbrechungsbereichen 11 versehenen Seitenwände ist aus Fig. 7 im einzelnen ersichtlich. Demnach sind diese Seitenwände - wie auch beim zuerst beschriebenen Ausführungsbeispiel - sandwichartig aufgebaut und bestehen aus dem Wandkörper 10 des Unterteils mit gitterartigen Durchbrechungsbereichen 11, einer außen an diesem Wandkörper anliegenden Filtermatte 3, welche die keimdichte, aber sterilisiergasdurchlässige Filterschicht bildet, um einer äußeren Halteplatte 4, die an der Außenseite der Filtermatte 3 anliegt und diese in Anlage mit der Außenfläche des Wandkörpers 10 hält und ebenfalls mit gitterartigen Durchbrechungsbereichen 41 versehen ist, die in Form und Anordnung den gitterartigen Durchbrechungsbereichen 11 des Wandkörpers entsprechen und damit also deckungsgleich sind. Die Halteplatte 4 ist wiederum seinem Unterkantenbereich und seinem Oberkantenbereich in entsprechende flache Nuten 16a, 18a eines unteren Stegs 16 und eines oberen Stegs 18 einrastbar ist, die außen seitwärts vorspringend am unteren und oberen Randbereich des Seitenwandkörpers 10 angeformt sind. Die Nut 16a ist als flache Dreiecknut ausgebildet, die eine schräge Sitzfläche für die entsprechend abgeschrägte Unterkante der Halteplatte 4 bildet, und die obere Nut 18a ist etwa als flache Rechtecknut ausgebildet, in die der abgerundete Oberkantenbereich der Halteplatte einrastbar ist. Die Halteplatte wird zunächst mit ihrer Unterkante auf die Sitzfläche der unteren Nut 16a aufgesetzt, und dann wird sie mit ihrem Oberkantenbereich an den Wandkörper angedrückt, so daß ihr Oberkantenbereich in die obere Nut 18a einrastet. Dabei sind die geometrischen Verhältnisse im Oberkanten- und Unterkantenbereich der Halteplatte 4 und den Nuten 16a und 18a in für den Durchschnittsfachmann leicht nachvollziehbarer Weise so gestaltet, daß die eingerastete Halteplatte 4 unter Vorspannung an der Filtermatte 3 anliegt und diese gegen den Wandkörper 10 andrückt.

Es versteht sich von selbst, daß die Gestaltung der Rastnuten 16a und 18a und der entsprechenden Kantenbereiche der Halteplatte 4 auch in anderer zweckmäßiger Weise ausgebildet sein könnte.

Mit Hilfe der rastbaren Halteplatten 4 können die beiderseitigen Filtermatten 3 von Zeit zu Zeit, also jeweils nach einer gewissen Anzahl von Sterilisiervorgängen, ersetzt werden.

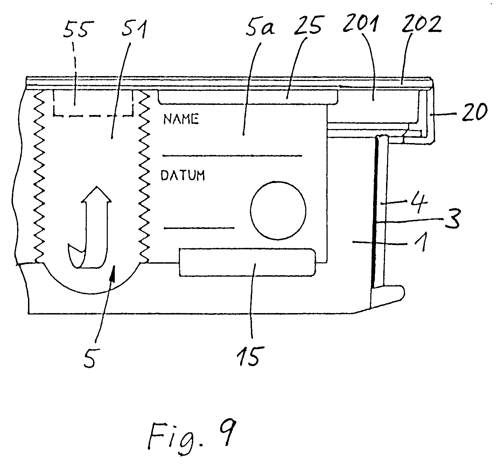

Die Seitenwände des Unterteils 1 endigen oben, wie aus den Fig. 7 und 8 ersichtlich ist, wieder in einem umlaufenden, nach oben weisenden Doppelstegprofil 12, in welches ein entsprechend komplementäres, nach unten weisendes Doppelstegprofil 22 am Unterseitenrandbereich eines Deckelkörpers 20 eingreift und damit zusammen einen keimdichten Verschluß herstellt.

Wie schon gesagt, ist bei dieser Ausführung der Deckel 2 zweiteilig ausgebildet und besteht, wie aus Fig. 8 ersichtlich ist, aus dem Deckelkörper 201, an dem das Doppelstegprofil 22 gebildet ist, und einem Arretierrahmen 202. Der Deckelkörper 201 und der Arretierrahmen 202 sind um eine gewisse Distanz relativ zueinander längsverschieblich. Wie Fig. 8 zeigt, sind die beiden Längsränder des plattenförmigen Deckelkörpers 201 in einer jeweils im seitlichen Randteil 20 des Arretierrahmens 202 gebildeten Nut 26 längsverschieblich geführt, die unterseitig durch einen durchgehenden oder vorzugsweise unterbrochenen, d.h. nur aus einer Reihe einzelner Nasen bestehenden, einwärts weisenden Steg 27 begrenzt ist. Der Arretierrahmen 202 ist zur Gewichts- und Materialeinsparung, wie aus Fig. 6 ersichtlich ist, nur als entsprechend dem Deckelkörperumfang verlaufender Rahmen ausgebildet, wobei vorteilhafterweise die Abmessungen der vom Arretierrahmen umschlossenen mittigen Aussparung und die Gestaltung der Innenumfangskante 20a des Arretierrahmens komplementär zur Außenform des Bodenbereichs des Unterteils 1 gewählt sind, so daß die vom Arretierrahmen 202 umschlossene Aussparung zusammen mit der Oberfläche des Deckelkörpers 201 eine Mulde darstellt, in welche eine weitere Box mit ihrem Unterteilbodenbereich einsetzbar ist und dann längs und quer verschiebsicher festgelegt ist. Auf diese Weise können mehrere Boxen zum Sterilisieren aufeinander gestapelt werden.

Wie Fig. 8 außerdem erkennen läßt, ist der obere, mit dem Doppelstegprofil 12 versehene Randteil jeder der beiden längeren Seitenwände des Unterteils 1 mit einer Anzahl einzelner, seitwärts nach außen vorspringenden Nasen 18a versehen, die jeweils eine gewisse begrenzte Länge haben. Ebenso ist der sich nach unten erstreckende Randteil 20 des Arretierrahmens 202 unten mit einer entsprechenden Anzahl einwärts weisender Vorsprünge 28 versehen, die etwa gleiche Längsausdehnung wie die Nasen 18a haben, wobei die Bemessung bzw. Passung so gewählt ist, daß die Vorsprünge 28 des Arretierrahmens 202 die Nasen 18a des Unterteils 1 unter einer gewissen elastischen Vorspannung und Verformung untergreifen, so daß dadurch das Doppelstegprofil 22 des Deckelkörpers 201 in straffe und damit gut dichtende Anlage mit dem Doppelstegprofil 12 des Unterteils gespannt wird. In Fig. 6 ist die Sterilisierbox mit dieser Position der beiden Deckelteile dargestellt, d.h. der Deckelkörper 201 ist mittels des Arretierrahmens 202 in seiner Schließstellung auf dem Unterteil verriegelt. In dieser Position der Deckelteile befinden sich an der hinteren Schmalseite des Deckelrandbereiches an Arretierrahmen 202 und Deckelkörper 201 gebildete Plombierbohrungen 28 in Überdeckung und ermöglichen das Sichern dieser verriegelten Position mittels einer Plombe, deren Vorhandensein dann auch anzeigt, daß das sterilisierte Behältnis bis zum Gebrauch ungeöffnet war.

Die Länge der Lücken zwischen den Nasen 18a des Unterteils 1 ist vorzugsweise wesentlich größer als die Länge der Vorsprünge 28 am Arretierrahmen 202, und es genügen beispielsweise drei über die Länge der Boxlängsseite verteilte Nasen 18a und Vorsprünge 28 mit jeweils etwa 1,0 bis 1,5 cm Länge. Die Verriegelung des Deckels 2 auf dem Unterteil 1 kann dadurch gelöst werden, daß der Arretierrahmen 202 um eine entsprechende Distanz relativ zum Deckelkörper 201 längsverschoben wird (siehe Pfeile in Fig. 6), wodurch der Deckel 2, nämlich der Deckelkörper 201 mit dem damit längsverschieblich verbundenen Arretierrahmen 202 vom Unterteil 1 abgehoben werden kann, indem die Vorsprünge 28 zwischen den Lücken der Nasen 18a hindurchbewegt werden.

Es versteht sich von selbst, daß miteinander zusammenwirkende Anschläge am Arretierrahmen 202 und am Deckelkörper 201 den notwendigen Längsverschiebeweg zwischen den beiden Deckelteilen beiderseits begrenzen. Trotzdem sind die beiden Deckelteile leicht zusammensetzbar und auch wieder auseinandernehmbar, indem sie an einer Schmalseite elastisch etwas auseinandergedrückt werden, um die miteinander zusammenwirkenden, den Verschiebeweg begrenzenden Anschläge aneinander vorbei zu bewegen, wonach dann der Deckelkörper 201 einfach in Längsrichtung aus dem Arretierrahmen 202 herausgezogen bzw. in diesen eingeschoben werden kann.

Wie aus Fig. 8 außerdem ersichtlich ist, kann der obere Randbereich der Halteplatte 4 an den Stellen, die der Lage der Nasen 18a und der Vorsprünge 28 bei verriegeltem Deckel 2 entsprechen, seinerseits mit seitwärts nach außen vorspringenden Nasen 42 ausgebildet sein, deren Außenkantenflächen mit den Innenkantenflächen der Vorsprünge 28 unter elastischer Vorspannung zusammenwirken, derart, daß die Vorsprünge 28 des Arretierrahmens 202, wenn dieser den Deckelkörper 201 im Zusammenwirken mit den Nasen 18a verriegelt, auch gleichzeitig die Halteplatten 4 sichert und vorzugsweise unter gewissem seitlichem Anpreßdruck am Wandkörper des Unterteils 1 hält. Damit wird irgendein unbeabsichtigtes Lösen der Halteplatten 4 während der Handhabung oder dgl. ausgeschlossen und damit die Keimdichtheit durch satte Anlage der Filtermatte 3 absolut gewährleistet. Wie aus Fig. 6 ersichtlich ist, sind die Halteplatten 4 an ihren vorderen und hinteren Stirnenden durch am Wandkörper 10 des Unterteils 1 angeformte leistenartige Vorsprünge 10a gegen jegliche Längsverschiebung gesichert.

Es versteht sich außerdem von selbst, daß die Nasen 18a sowie 42 und/oder die Vorsprünge 28 im Bereich der zusammenwirkenden Flächen am entsprechenden Längsende etwas rampenförmig ausgebildet sind, um ein leichtes Aufeinanderschieben der miteinander zusammenwirkenden Flächen zu ermöglichen.

Fig. 9 zeigt wieder das an der vorderen Stirnseite der geschlossenen und sterilisierten Sterilisierbox angeordnete Sicherungsetikett 5, mit Aufreißlasche 51 das ein Entriegeln des Deckels ohne Zerstörung des Sicherungsetiketts verhindert und infolgedessen auch durch seinen unbeschädigten Zustand anzeigt, daß der Deckel seit dem Sterilisiervorgang bis zum Öffnen der Sterilisierbox zwecks Entnahme des darin enthaltenen Instruments zum Gebrauch noch nicht geöffnet worden ist, und daß folglich das darin enthaltene Instrument noch steril ist.

Wie aus Fig. 9 ersichtlich ist, greift das Sicherungsetikett mit oberen und unteren Randbereichen seiner beiderseits der Aufreißlasche 51 befindlichen Etikettabschnitte 5a in an vorspringenden Leisten 15 des Unterteils 1 und 25 des Deckelkörpers 201 gebildete Nuten ein, die an den außenliegenden Enden geschlossen sind, so daß das Sicherungsetikett nicht unbeschädigt seitwärts aus diesen Nuten herausschiebbar ist. Außerdem ist am Arretierrahmen 202 des Deckels eine nach unten ragende, hinter den oberen Randbereichen der Aufreißlasche 51 greifende Nase 55 angeformt, die bei intaktem Sicherungsetikett verhindert, daß der Arretierrahmen, wie in Fig. 1 durch Pfeile sichtbar gemacht ist, nach vorne in Lösestellung verschoben werden kann, da ja die oberen Randbereiche des Sicherungsetiketts beiderseits durch die Leisten 25 des Deckelkörpers 201 festgehalten wird. Diese Nase 55 stellt zugleich auch einen der den Verschiebeweg des Arretierrahmens relativ zum Deckelkörper begrenzenden Anschläge dar, der mit einer zurückgesetzten Gegenfläche an der Außenkante des Deckelkörpers zusammenwirkt, während der entsprechende Begrenzungsanschlag am anderen Deckelende zweckmäßig durch eine an der Oberseite des Deckelkörpers gebildete kleine Nase 29 gebildet sein kann, die mit der Innenumfangskante 20a des Arretierrahmens zusammenwirkt.

Aus Fig. 1 ist ersichtlich, daß der flach auf der Oberseite des Deckelkörpers 201 aufliegende Rahmenkörper des Arretierrahmens 202 an seinen Längsseiten eine Reihe von Durchbrüchen 20b aufweist, die sich jeweils über den Vorsprüngen 28 der Randteile 20 und den Stegabschnitten 27 befindet, was die Fertigung des Arretierrahmens im Kunststoffspritzgußverfahren erleichtert, weil auf diese Weise Hinterschneidungen vermieden werden können (die Aussparungen 20b sind jeweils etwas länger und breiter als die Vorsprünge 28 bzw. die Stegabschnitte). In diesem Zusammenhang ist darauf hinzuweisen, daß diese Aussparungen 20b in Fig. 8 nicht dargestellt sind, weil Fig. 8 eine vereinfachte Darstellung dergestalt wiedergibt, daß die Vorsprünge 28 und die Stegabschnitte 27 sich an der gleichen Position befinden, was aber in der praktischen Ausführung nicht der Fall ist, vielmehr sind in der praktischen Ausführung die Vorsprünge 28 und die Stegabschnitte 27 entlang des betreffenden Längsrandes gegeneinander versetzt angeordnet, um den ebengenannten fertigungstechnischen Vorteil zu erreichen.

## Patentansprüche

1. Mehrfach sterilisierfähiges Behältnis für einzelne medizinische und chirurgische Bestecke, Instrumente und Geräte in Form einer steifen Box aus sterlilisiertemperaturbeständigem Kunststoff, bestehend aus einem Unterteil (1) mit Seitenwenden und einem darauf aufsetzbaren und keimdicht abschließenden Deckel (2), wobei in Unterteilseitenwandbereichen mit einer keimdichten, jedoch für Sterilisierdampf bzw. Sterilisiergas durchlässigen Filterschicht (3) abgedeckte Wanddurchbrüche (11) vorgesehen sind, dadurch gekennzeichnet, daß der Deckel (2) zweiteilig ausgebildet ist und einen Deckelkörper (20), dessen Rand unterseitig mit einem umlaufenden Dichtungsprofil (22) versehen ist, das mit einem im Oberkantenbereich der Unterteilseitenwände gebildeten komplementären Dichtungsprofil (12) zusammenwirkt, und einen Arretierrahmen (202) aufweist, der den Deckelkörper (201) mindestens teilweise entlang zweier gegenüberliegender Randkanten umgreifende Randteile (20) aufweist und parallel zu den umgriffenen Deckelkörperrandkanten um eine gewisse Distanz verschiebbar mit dem Deckelkörper verbunden ist, und daß am Oberkantenbereich der den umgriffenen Deckelkörperrandkanten entsprechenden Unterteilseitenwände (10) seitwärts nach außen vorspringende Nasen (18b) und am Arretierrahmen (202) dazu etwa komplementäre, einwärts seitlich vorspringende Vorsprünge (28) gebildet sind, die durch Verschiebung des Arretierrahmens (202) mit Bezug auf den durch das Unterteil (1) aufgesetzten Deckelkörper (201) zwischen einer die Nasen (18b) des Unterteils untergreifenden Arrtierstellung und einer mit Bezug auf die Nasen (18b) des Unterteils versetzten Lösestellung bewegbar sind, und mit einem den Arrtierrahmen in seiner Arrtierstellung gegen eine Lösebewegung sicherenden Sicherungsorgan (5, 28), das zerstörbar ist, um das Öffnen des Behältnisses zu ermöglichen.

2. Behältnis nach Anspruch 1, dadurch gekennzeichnet, daß die Randteile (20) des Arretierrahmens (202) jeweils eine Führungsnut (26) zur Aufnahme der betreffenden Deckelkörperrandkante aufweisen, die durch die Deckelkörperrandkante oben und unten übergreifende Leistenabschnitte (27) gebildet ist.

3. Behältnis nach Anspruch 1 oder 2, dadurch gekennzeichnet, daß am Arretierrahmen (202) und am Deckelkörper (201) miteinander zusammenwirkende, den relativen Verschiebeweg von Arretierrahmen und Deckelkörper beiderseits begrenzende Anschläge (29, 55) vorgesehen sind.

4. Behältnis nach einem der Ansprüche 1 bis 3, dadurch gekennzeichnet, daß der Arretierrahmen (202) einen flachen, auf dem Randbereich des Deckelkörpers (201) aufliegenden Rahmenkörper mit den daran angeformten, die betreffenden Deckelkörperrandkanten umgreifenden Randteilen (20) aufweist, und daß der Rahmenkörper im Bereich der einwärts weisenden Randteilvorsprünge (28) sowie im Bereich von die Deckelkörperrandkante unten übergreifenden Leistenabschnitten (27) mit Durchbrüchen (20b) versehen ist.

5. Behältnis nach einem der Ansprüche 1 bis 4, dadurch gekennzeichnet, daß der flache, auf der Oberseite des Deckelkörpers (201) aufliegende Rahmenkörper des Arretierrahmens (202) mit seinen Innenumfangsrand (20a) eine unten durch die Oberseite des Deckelkörpers (201) begrenzte Mulde umschließt, die komplementär zum Bodenbereich des Unterteils (1) geformt ist.

6. Mehrfach sterilisierfähiges Behältnis für einzelne medizinische und chirurgische Bestecke, Instrumente und Geräte in Form einer steifen Box aus sterilisiertemperaturbeständigem Kunststoff, bestehend aus einem Unterteil (1) mit Seitenwänden und einem darauf aufsetzbaren und keimdicht abschließenden Deckel (2), wobei in Unterteilseitenwandbereichen mit einer keimdichten, jedoch für Sterilisierdampf bzw. Sterilisiergas durchlässigen Filterschicht (3) abgedeckte Wanddurchbrüche (11) vorgesehen sind, dadurch gekennzeichnet, daß am Unterteil (1) und am Deckel (2) im Bereich einer der vier Seiten des Behältnisses angeordnete, miteinander zusammenwirkende Formschlußelemente (13, 23), die bei geschlossem Deckel ein Abheben derselben vom Unterteil verhindern, und an der gegenüberliegenden Seite des Behältnisses federelastisch miteinander zusammenwirkende Rastelemente (14, 24) vorgesehen sind, und daß an Unterteil und Deckel Haltemittel (15, 25) vorgesehen sind, die das Einsetzen eines die Rastelemente (14, 24) verdeckenden Sicherungsorgans (5) während des Schließens des Deckels ermöglichen und bei geschlossenem Deckel das Sicherungsorgan (5) nach allen Richtungen arretieren, wobei das Sicherungsorgan zerstörbar ist, um den Zugang zu den Rastmitteln (14, 24) zum Öffnen des Behältnisses zu ermöglichen.

7. Behältnis nach Anspruch 6, dadurch gekennzeichnet, daß die Formschlußelemente einen oder mehrere am oberen Rand des Unterteils (1) einwärts vorragende leisten- oder noppenartige Vorsprünge (13) sowie ein oder mehrere an der Deckelunterseite angeordnete, schräg nach unten und auswärts orientierte leisten- oder rippenartige, die Vorsprünge (13) hintergreifende Gegenelemente (23) aufweisen.

8. Behältnis nach Anspruch 6 oder 7, dadurch gekennzeichnet, daß die Rastelemente eine am Deckel angeordnete, nach unten vorspringende Lasche (24) mit einer angeformten Rastnase und ein am Randbereich des Unterteils angeordnetes, beim Einrasten von der Rastnase der Rastlasche (24) hintergriffenes Gegenelement (14) aufweisen.

9. Behältnis nach einem der Ansprüche 1 bis 8, dadurch gekennzeichnet, daß das Sicherungsorgan durch eine als Etikett verwendbare Sicherungsplatte (5) aus Karton oder dgl. mit einem vorgestanzten bzw. perforierten Aufreißbereich (51) gebildet ist.

10. Behältnis nach den Ansprüchen 1 und 9, dadurch gekennzeichnet, daß die Sicherungsplatte (5) an einer mit Bezug auf die umgriffenen Deckelkörperrandkanten quer verlaufenden Seite angeordnet und in einen mittigen Aufreißbereich (51) und zwei beiderseits derselben befindliche Abschnitte (5a) unterteilt ist, und daß an der betreffenden Unterteilseitenwand Nutleisten (15) zur Aufnahme der Unterkantenbereiche der seitlichen Sicherungsplattenabschnitte (5a) und am Deckelkörper die Oberkantenbereiche dieser Sicherungsplattenabschnitte aufnehmende Nutleisten (25) gebildet sind und Anschläge gegen eine seitliche Verschiebbarkeit der Sicherungsplatte (5) vorgesehen sind, und daß der Arretierrahmen (202) einen den Aufreißbereich (51) der Sicherungsplatte (5) hintergreifende Nase (55) aufweist, die eine relative Verschiebung des Arretierrahmens zum Deckelkörper aus seiner Arrtierstellung in die Lösestellung ohne Zerstörung der Sicherungsplatte sperrt.

11. Behältnis nach den Ansprüchen 1 und 6, dadurch gekennzeichnet, daß die Sicherungsplatte (5) einen mittig vertikal verlaufenden Aufreißbereich (51) aufweist, der die ebenfalls mittig an Unterteil bzw. Deckel angebrachten Rastelemente (14, 24) verdeckt, und daß die Haltemittel (15, 25) für die Sicherungsplatte (5) einander zugewandte Einstecknuten sind, die beiderseits des Aufreißbereichs an Unterteil und Deckel gebildet sind und außerdem die Sicherungsplatte seitwärts beiderseits arretierende Halteelemente aufweisen.

12. Behältnis nach einem der Ansprüche 1 bis 11, dadurch gekennzeichnet, daß die Filterschicht (3) jeweils in Form einer außenseitig an den betreffenden Seitenwänden des Unterteils (1) anliegende Filtermatte gebildet ist und daß die Filtermatte mittels einer Halteplatte (4) mit den Wanddurchbrüchen (11) entsprechenden Durchbrüchen (41) in ihrer Lage gehalten wird, die lösbar an der betreffenden Seitenwand befestigt ist.

13. Behältnis nach Anspruch 12, dadurch gekennzeichnet, daß jede Halteplatte (4) mit ihren Unterkanten- und Oberkantenbereichen in entsprechende Haltenuten (16a, 18a) von an der Seitenwand (10) gebildeten leistenartigen Vorsprüngen (16, 18) einrastbar ist.

14. Behältnis nach Anspruch 13, dadurch gekennzeichnet, daß die Halteplatte (4) an ihren beiden Stirnenden durch an der betreffenden Unterteilseitenwand (10) angeformte leistenartige Vorsprünge (10a) oder dgl. gegen Längsverschiebung gesichert ist.

15. Behältnis nach den Ansprüchen 1 und 12, dadurch gekennzeichnet, daß die Halteplatte (4) an ihrem Oberkantenbereich mit einer Anzahl seitlich auswärts ragender Nasen (42) versehen ist, deren Lage der Position der Nasen (18b) der betreffenden Unterteilseitenwand (10) entspricht, und daß die einwärts weisenden Vorsprünge (28) der Seitenteile (20) des Arretierrahmens (202) mit ihren inneren Kantenflächen in der Arretierposition mit den äußeren Kantenflächen der Halteplattennasen im Sinne der Arretierung der Halteplatten in Anlage an der betreffenden Unterteilseitenwand (10) zusammenwirken.
